# EUROPEAN PATENT APPLICATION

(11) **EP 3 992 278 A2**
(43) Date of publication of application: **04.05.2022**
(21) Application number: 20877973.6
(22) Date of filing: 26.11.2020
(51) Int. Cl.: C12N 1/12, C12M 1/00, C12M 1/36, C12M 1/42, C12M 3/02, A01G 33/00

(54) **METHOD FOR CULTIVATING MICROALGAL BIOMASS AND APPARATUS FOR THE IMPLEMENTATION THEREOF**

(30) Priority: 17.10.2019 RU 2019130022
(71) Applicant: Grabarnik, Vladimir Efimovich, Moscow 170002 (RU); Karelin, Nikolay Viktorovich, Tver, 170002 (RU)
(72) Inventor: Grabarnik, Vladimir Efimovich, Moscow 170002 (RU); Karelin, Nikolay Viktorovich, Tver, 170002 (RU)
(74) Representative: Berggren Oy
(86) International application number: PCT/RU2020/050351
(87) International publication number: WO 2021/076021

(57) **Abstract**

The invention relates to a process and equipment for cultivating and producing a biomass from microalgae, primarily plankton. A microalgal biomass is cultivated in a single bioreactor chamber in the shape of a vertically oriented parallelepiped. The culture mixture is illuminated by artificial light sources mounted on the inner side of one of the broad walls of the bioreactor chamber in horizontal rows throughout the height of the bioreactor chamber. The culture mixture is illuminated cyclically. Spray heads are mounted on the inner side of the opposite wall of the bioreactor chamber so that 4 spray heads are arranged symmetrically around each artificial light source. The pH value of the culture mixture is maintained in a range of 8.5-9.5 by the addition thereto of a lactic acid bacteria-containing solution at the beginning of each light cycle in an amount of 1-3 ml per litre of culture mixture, said solution having a pH value selected in a range of 4.0-5.0.

## Description

### Field of the invention

The group of inventions relates to the field of biotechnology, namely, to a process and equipment for cultivating and producing a biomass from microalgae, primarily plankton, for example, chlorella, and could be used in microbiological and pharmaceutical industry.

### Background

Microalgal biotechnology has arisen from the researches related to three different fields: space, submarines and bomb shelters. These researches have been aimed at development of closed life support systems. Such systems can be used in the conditions without bleeding of carbonic acid gas formed as a result of human processes, and also where food sources in the form of protein, carbohydrates and lipids are required. Microalgal biotechnologies use the fundamental property of plants - ability of photosynthesizing cells to fix CO₂ from atmosphere and transform it into different substances (biomass) with oxygen emission to atmosphere. Since microalgae comprise a complete set of micro- and macroelements, including all essential amino acids, vitamins B₁, B₂, B₆, and B₁₂, magnesium, iron, zinc, lean fats and vitamin A (beta-carotene), their use enables to support activities of living beings at the molecular level.

The prior art discloses a method of producing microalgal biomass, in particular, chlorella suspension, which involves filling of medium and chlorella strain stock culture into a bioreactor in the form of clear container, culture liquid illumination by artificial light source while maintaining the required culture liquid temperature in the bioreactor, extracting the chlorella biomass as a target product (patent for an invention RU 2176667, IPC C12N 1/12, C12M 3/00, publ. 10.12.2001).

The above method is implemented in the apparatus representing the clear containers illuminated by artificial light source. The containers are spaced some distance apart on the framework tray around a light source, while the light source placed on the framework is configured to move vertically to the tray.

The first day of chlorella cultivation the light source is in the upper limit position, the second day it is lowered to 2/3 distance from the tray, and the third day - to 1/3 distance from the tray, and the light source remains in this position till the end of cultivation, that enables to maintain optimal illumination and temperature of the suspension within the whole process. Duration of daily illumination in the process of microalgae cultivation is 20-22 hours.

The main disadvantages of the known method implemented in the known apparatus are low productivity and poor quality of the ready product, that prevents from using these method and apparatus in the food industry.

The prior art discloses a method for continuous producing of planktonic algae, primarily food chlorella, including mineral medium preparation, adding of microalgae strain stock culture, filling of the produced culture mixture into the bioreactor in the form of light-transmitting chambers, illumination of the culture mixture by vertically arranged artificial light sources, extraction of the produced suspension to the collecting tank, extraction of the produced precipitated biomass as a target product (patent for an invention RU 2571939, IPC C12M 1/00, C12M 3/02, C12N 1/12, 3/02, A01G 33/00, publ. 27.12.2015). The known method could be implemented in the apparatus comprising two suspension chambers placed on the framework, illumination lamps, tanks for nutrient solution preparation and for ready suspension collection and storage, which are connected to the chambers by pipelines. Both microalgal suspension chambers are arranged on the framework to change the distance between them and are interconnected by a pipeline in the lower part of the vertical walls to balance suspension volume in both chambers. Each suspension chamber has two drain holes, one of them is in the lower part of the side wall for draining the ready chlorella suspension and its outflowing through the pipeline to the suspension collection and storage tank, and the second hole is in the bottom surface. One of the suspension chambers is configured to cultivate a stock culture and ensure a single biotechnological process. Illumination lamps in each chamber are arranged eccentrically relative to its longitudinal axis. An illumination lamp between the chambers is fixed on the separate frame independently of the framework and chambers and it is configured to move freely and dismount when changing-over to solar illumination. Nutrient solution tank is located above the chambers' level and has two holes in the vertical side wall, the first one is on the 0.5 volume level, and the second one - in the bottom layer and it is adapted to connect pipelines for draining the nutrient solution to aquariums.

The disadvantages of the known method for continuous producing of planktonic algae and apparatus for the implementation thereof are poor quality of ready product and low specific productivity that prevents from using the known method and apparatus in the food industry.

The prior art discloses a method for producing microalgal biomass, in particular, food chlorella suspension, including chlorella cultivation in automatic transparent bioreactor while illuminating the culture mixture by artificial light sources within 4 light cycles, each of them consisting of 10 hours of illumination and subsequent 2 hours of no illumination, during illumination period the culture mixture temperature is maintained withing the range of 26-30°C, and during the period of no illumination - within the range of 24-26°C, extraction of the produced suspension to the natural precipitation tank within 30 days (patent for an invention RU 2662974, IPC C12N 1/12, C12M 3/00, C12M 1/00, 3/02, C12R1/89, publ. 31.07.2018).

Notwithstanding the fact that implementation of the known method enables to improve quality of ready product as compared to the above methods known in the art, practically it fails to achieve such specific productivity measures which could ensure effective use of this method in the food industry.

The closest prior art to the claimed group of inventions is the invention disclosed in the patent RU 2540011 (IPC C12M 1/00, C12M 3/02, A01G 33/00, publ. 27.01.2015).

The said patent discloses an apparatus for chlorella cultivation comprising the system of chlorella bioreactors in the form of transparent horizontally oriented chambers connected to the ready suspension tank by the ready suspension rundown line, carbon dioxide solution reactor connected at the outlet with chlorella bioreactors, nutrient solution preparation plant connected at the outlet with chlorella bioreactors and carbon dioxide solution reactor, pumps and shutoff and control devices. Illumination lamps in the form of electric lamps are equipped with the cooling system and are installed inside the vessels of chlorella bioreactors. Stationary spray heads are mounted inside the chlorella bioreactor vessels, and these heads are connected at the inlets with the washing fluid preparation system. Mixing, control, washing and draining devices are mounted and connected under the chlorella bioreactors, which are equipped with washing fluid drain lines to a drainage and connected at the inlet with the ready suspension tank.

Method for cultivating microalgae is implemented as follows. Water is preliminarily purified from unintended impurities. Then, purified and heated water enters the plant for of nutrient solution preparation, which is used in microalgae cultivation in the chlorella bioreactors, and also in the process of carbon dioxide production in the lactic-acid bacteria bioreactor. Nutrient solution for the lactic-acid bacteria bioreactor is supplied by a pump and controlled by a flow meter and controller. Nutrient solution for producing algal suspension the chlorella bioreactors is supplied through the pressure line also by a pump through a controller operated solenoid valve. Algal stock culture is supplied from the ready suspension tank by the pump through the corresponding solenoid valve. Carbon dioxide solution is dosed by the carbon dioxide solution pump from the lactic-acid bacteria bioreactor to the chlorella bioreactors through the solenoid valve of lactic-acid bacteria solution filling to the chlorella bioreactor and is controlled by the flow meter and controller. Chlorella bioreactor chambers are illuminated by the artificial light sources mounted vertically inside the chamber vessels. Illumination time and periodicity are controlled by the controller. Illumination lamps ensure simultaneous illumination and maintaining the microclimate in algal suspension. After producing the ready product the chlorella bioreactors are drained through the ready product drain valve by means of the pump to the ready suspension tank, then, the bioreactor chambers are washed by the spray heads system. The spray heads are fed by means of the pump station with the washing fluid preparation system for the chlorella bioreactors, bioreactor chambers are drained through the ball cock.

The apparatus and method for chlorella cultivation known in the patent RU 2540011 also have some disadvantages. In particular, implementation of the group of inventions of the said patent does not allow to achieve high measures of specific productivity mainly due to the fact that the apparatus occupies considerable production area since the bioreactor chambers are horizontally oriented, and also due to long intervals between chlorella cultivation cycles and inconvenience in servicing.

The described known methods for producing food chlorella and apparatuses for the implementation thereof have certain advantages as compared to the methods apparatuses based on surface illumination of the stock solution. However, all known methods for producing microalgae and apparatuses for the implementation thereof, including the closest prior arts, have substantial disadvantages. First of all, they can include insufficient efficiency of processes in the bioreactor chamber, considerable areas occupied, and high costs per ready product unit, and also limitation of the apparatus productivity caused by necessity to make pauses in operation for cleaning the lamps used as light sources, and by inconvenience in servicing.

### Disclosure of the Invention

Technical problem, for solving of which the claimed group of inventions is intended, is creation of such method and apparatus for the implementation thereof, which could enable to produce high quality microalgal suspension with high specific productivity.

In contrast to the technical solutions known in the prior art this problem is solved in the claimed group of inventions by organizing optimal process conditions from the perspective of increasing the apparatus specific productivity, reducing the occupied production areas and improving convenience in servicing. Such conditions, when implementing the claimed group of inventions, are achieved as resulting from creation of efficient process of microalgae cultivation, as due to development of principally new equipment of the apparatus.

The claimed technical solutions (method and apparatus) are aimed at solving the said problem and achieving the technical result consisting in improving the ready product quality and specific productivity, and also in reducing the materials consumption.

In terms of the method the claimed technical result is achieved due to the fact that in the method for producing microalgae, in particular, food chlorella suspension, where the medium, including the nutrients N, P, Fe, C 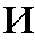 , Co, and chlorella strain stock culture are filled into the bioreactor in the form of transparent container, culture liquid is illuminated by artificial light source while maintaining the required culture liquid temperature in the bioreactor, and using the carbon dioxide as carbon nutrition, produced organically due to lactic-acid bacteria metabolism, extraction of chlorella biomass as a target product, the prepared mineral medium with the following composition is used:

| | |
|---|---|
| ammonium nitrate (34% solution) | - 0.14 ml |
| ammophos (15% solution) | - 0.10 ml |
| ferrous chloride (1% solution) | - 0.15 ml |
| cobaltous nitrate (0.1% solution) | - 0.10 ml |
| copper sulphate (0.1% solution) | - 0.10 ml |
| potable water | - 1000 ml |

- biomass is cultivated in a single chamber in the shape of a vertically oriented parallelepiped,
- illuminating the culture mixture by artificial light sources mounted on the inner side of one of the broad walls of the bioreactor chamber in horizontal rows throughout the height of the bioreactor chamber,
- illuminating the culture mixture cyclically,
- during all the cycles of microalgae cultivation pH value of the culture mixture is maintained in a range of 8.5-9.5 by the addition thereto of a lactic acid bacteria-containing solution at the beginning of each light cycle in an amount of 1-3 ml per litre of culture mixture, said solution having a pH value selected in a range of 3.5-4.0.

The claimed technical result is achieved due to the whole set of essential features stated in the independent claim 1 characterizing the subject matter "method".

Use of mineral medium with the specified composition for microalgae cultivation comprising in 1000 ml of purified potable water 0.14 ml of ammonium nitrate (34% solution), 0.10 ml of ammophos (15% solution), 0.15 ml of ferrous chloride (1% solution), 0.10 ml of cobaltous nitrate (0.1% solution), 0.10 ml of copper sulphate (0.1% solution), in combination with cyclic illumination of the culture mixture, which PH during all the cycles of microalgae cultivation is maintained in a range of 8.5-9.5 by the addition thereto of a lactic acid bacteria-containing solution at the beginning of each light cycle in an amount of 1-3 ml per litre of culture mixture, said solution having a pH value selected in a range of 3.5-4.0, enables to ensure the most favorable conditions for microalgae growth, and thus, to improve qualitative characteristics of the ready product and specific productivity.

The said set of essential features enables to produce high quality product based on Chlorella vulgaris strain, in particular, Detox Urban Drink with living chlorella, which is composed only of not heat treated living cells of Chlorella Vulgaris GKO strain, nutrient solution, where they have grown, composed of lactic-acid bacteria (Lactobacillus), chlorella metabolites (useful substances, which chlorella produces as grows) and pure artesian water subjected to special multistage treatment.

According to the composition analysis of Detox Urban Drink with living chlorella, produced on the basis of Chlorella vulgaris GKO strain, carried out by Wessling laboratory, there have been revealed 13 vitamins essential for human organism, 12 different minerals. The drink amino acid composition comprises 18 components, fatty acid composition - 12. A great number of elements contained in the drink are also antioxidants.

Detox Urban Drink comprises the following fatty acids: arachidonic (omega-6), linoleic (omega-6), docosahexaenoic (omega-3), alpha-linoleic (omega-3), linoleic, palmitic, oleic (omega-9), palmitoleic, stearic, myristic, pentadecanoic and erucic.

Detox Urban Drink comprises antioxidants of the carotenoid group (beta-carotene), polyphenols - lutein, catechin, epicatechin, epigallocatechin gallate, quercetin, vitamins A, E and C, and also minerals-antioxidants - selenium, copper, zinc, manganese.

Maintaining the claimed pH values of culture mixture and lactic acid bacteria-containing solution (Lactobacillus) during microalgae cultivation ensures viability of microalgae cells and preservation of their useful properties. Disturbance of cultivation conditions causes degradation of algae monospecies culture or contamination with weed bacteria, that directly results in loss of functional properties of the ready product.

The closed process of microalgae cultivation excludes a probability of contaminations and pathogens ingress into the bioreactors. However, even a short-time failure of parameters during the production cycle could result in serious problem, since each chlorella cell dividing twice per day is subjected to natural mutations, and its medium is nutritive for a number of weed bacteria.

The claimed method of the dependent claim 2 could be supplemented by the fact that the culture mixture is illuminated in the automatic bioreactor within four successive cycles, each of them consisting of 10 hours of illumination and subsequent 2 hours of no illumination with artificial light sources being Off, and during illumination period the bioreactor temperature is maintained withing the range of 26-30°C, and during the period of no illumination - within the range of 24-26°C. Illumination of the culture mixture in said mode enables to create the most optimal conditions for intensive growth and multiplication of microalgae cells. In such conditions optical density of microalgal culture solution reaches (1,4-1,8) D values (specified optical density). Moreover, the whole process is carried out subject to the sanitary conditions which are to be maintained at food production.

Shifting of temperature conditions to any side results in mutation of microalgae cells, and, as a consequence, in degradation of ready product quality and decrease of specific productivity.

Illumination of culture mixture within four successive cycles according to the dependent claim 2 has additive effect on improving the quality of produced microalgal suspension and specific productivity. We have used such illumination conditions before (patent RU 2662974). However, use of such conditions in combination with the claimed method techniques enables to create the most optimal conditions for microalgae cultivation, that in turn ensures the higher performance of the implemented process.

The claimed method of claim 3 could be supplemented by the fact that the culture mixture is filled into the bioreactor chamber above any horizontal row of light sources by a quantity equal to half of their center to center distance heightwise.

Ability to implement the invention of the dependent claim 3 enables to vary the bioreactor chamber filling in case of necessity to produce the specified amount of ready product. Filling the culture mixture into the bioreactor chamber above any horizontal row of light sources by a quantity equal to half of their center to center distance heightwise enables to vary the effective volume of the bioreactor chamber to produce the required amount of ready product with high specific productivity of the apparatus and high quality of the product.

In terms of device the claimed technical result is achieved due to the fact that in the microalgal biomass cultivation apparatus comprising successively mounted bioreactors in the form of transparent horizontal chambers, equipped with mixing means, connected to the ready microalgal biomass tank by a rundown line, the lactic-acid bacteria bioreactor connected at the outlet with microalgae bioreactors, medium preparation unit connected at the outlet with the microalgae bioreactors and lactic-acid bacteria solution bioreactor, artificial light sources in the form of electric lamps equipped with the cooling system, spray heads connected with the washing fluid preparation system, pumps and shutoff and control devices, microalgae bioreactor chamber is in the shape of a vertically oriented transparent parallelepiped, artificial light sources are mounted on the inner side of one of the broad walls of the chamber in horizontal η rows, perpendicular to it, throughout the height of the chamber, spray heads are mounted on the inner side of the opposite wall of the bioreactor chamber so that 4 spray heads are arranged symmetrically around each artificial light source.

The claimed technical result is achieved due to the whole set of essential features stated in the independent claim 4 characterizing the subject matter "device".

Bioreactor chamber in the shape of a single vertically oriented parallelepiped with artificial light sources mounted on the inner side of one of the broad walls of the chamber in horizontal η rows throughout the height of the chamber, and also mounting of the spray heads on the inner side of the opposite wall of the bioreactor chamber so that 4 spray heads are arranged symmetrically around each artificial light source enables to optimize the design of the apparatus itself so that it in turn results in optimal arrangement of microalgae cultivation processes ensuring producing the high quality ready product with high specific productivity, while minimizing materials consumption, occupied area and improving convenience in servicing. Chamber in the shape of a vertically oriented parallelepiped with several horizontal rows of artificial light sources mounted on one of its broad walls enables not only to minimize the area occupied by the apparatus, i.e. eventually increase specific productivity, but also to produce the specified amount of product in one and the same chamber depending on its filling level.

The claimed invention of the dependent claim 5 could be supplemented by the fact that the apparatus is equipped with programmable automatic system for control and monitoring the set parameters of temperature, volume, dosage, loading, unloading of solution and ready product.

The apparatus for implementation of the claimed method of the dependent claim 6 could comprise two and more bioreactors. Such apparatus arrangement enables to increase volume of high quality ready product manyfold as compared to the known solutions.

In general, the claimed group of inventions enables to improve productivity and ready product quality, and also serviceability, operational safety, efficiency of the lamp cooling system by effective equipment of the apparatus, enabling to ensure automatic production of microalgal suspension practically excluding necessity for manual labour in the apparatus operation.

### Brief description of drawings

The essence of the claimed group of inventions is supported by the drawings (Fig. 1 - Fig. 3). Fig. 1 illustrates a longitudinal section of the apparatus general view, Fig. 2 - section of the bioreactor chamber, Fig. 3 - section of the bioreactor chamber in plan view, Fig.

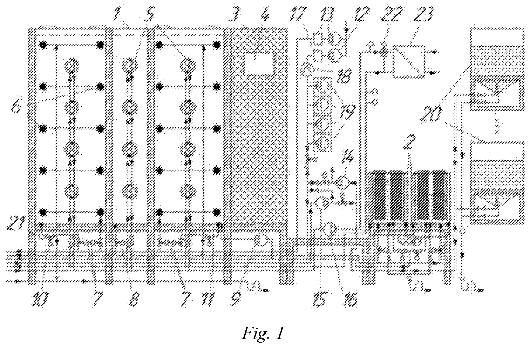

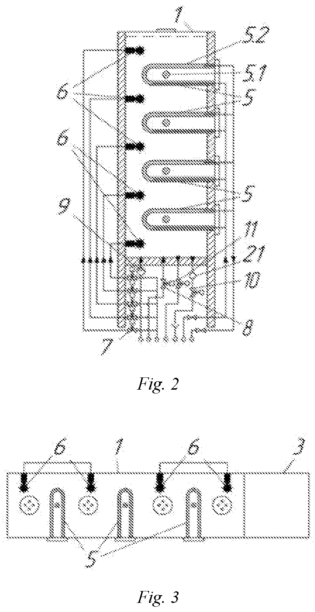

In accordance with the presented drawings (Fig. 1 - Fig. 3) the apparatus comprises:
1 - bioreactor chamber,
2 - lactic-acid bacteria solution bioreactor,
3 - I&C board,
4 - process controller,
5 - immersion lamp, where
   5.1 - electric lamp,
   5.2 - lamp shell with flow channel,
6 - spray heads,
7 - solenoid valves for spray heads control,
8 - solenoid valve for stock culture/medium supply,
9 - peristaltic dosing pump for lactic-acid bacteria solution supply,
10 - motorized drainage valve,
11 - motorized ready product drain valve,
12 - treated source water pump,
13 - pump station with the washing fluid preparation system,
14 - stock culture pump,
15 - ready product pump,
16 - lamp cooling system circulation pump,
17 - direct-flow water heater,
18 - medium flow meter,
19 - nutrient solution preparation plant,
20 - ready product tank,
21 - bioreactor level transducer,
22 - three-way motorized control valve of the lamp cooling circuit,
23 - lamp cooling circuit heat exchanger.

### Embodiment of the Invention

The inventive method is implemented in the apparatus of the claimed design.

The apparatus operates as follows.

The apparatus is located in a translucent room, that ensures additional illumination or complete change-over to solar illumination pf algal suspension, where air conditioning system without atmospheric air admixture is used. Devices ensuring such processes as dosing, lighting, mixing, measuring, controlling, washing, drainage and emptying are located in individual I&C board 3.

Feed preliminarily purified from unintended impurities is supplied by the pump 12 to the direct-flow water heater 17, then - to the nutrient solution preparation plant 19. During the above steps the nutrient solution is prepared, which is divided into two production lines and used in the process of cultivation in the algal suspension bioreactor and also in producing the lactic-acid bacteria solution in the lactic-acid bacteria solution bioreactor 2.

Nutrient solution for the lactic-acid bacteria solution 2 is supplied by the pump 12 and controlled by the flow meter 18 and controller 4. Nutrient solution for producing algal suspension the bioreactor is supplied through the pressure line also by the pump 12 through the controller 4 operated solenoid valve 8. Bioreactor medium level is controlled by the level transducer 21 and controller 4 mounted in the board 3. Microalgal stock culture is supplied from the ready suspension tank 20 by the pump 14 through the stock culture filling solenoid valve 8 and controlled on the principle of medium supply. Lactic-acid bacteria solution supply is dosed by the dosing pump 9 with fixed capacity from the lactic-acid bacteria bioreactor 2 to the bioreactor 1 and is time controlled by the controller 4 mounted in the board 3. The bioreactor 1 is illuminated by the lamps 5 with fluid cooling jacket mounted inside the vessels. Each lamp is in the form of electric lamp 5.1 equipped with additional transparent shell 5.2 enveloping the electric lamp 5.1 case using the flow channel, wherein inlet and outlet are interconnected by the cooling system heat exchanger 23, cooling fluid supply line and rundown line. Cooling fluid is circulated by the pump 16 and controlled by three-way motorized control valve 22. Medium illumination time and periodicity are controlled by the controller 4 mounted in the board 3. Illumination lamps 5 ensure simultaneous illumination and maintaining the microclimate in microalgal suspension. After producing the ready product, the bioreactor 1 is drained through the ready product motorized ball cock 11 by means of the pump 15 to the ready suspension tank 20, then, the sections are washed by the spray heads 6 system. The spray heads are fed by means of the pump station 13 with the washing fluid preparation system for the chlorella bioreactors, bioreactor chamber is drained through the motorized ball cock 10.

The system of stationary spray heads connected at the inlets with the washing fluid preparation system mounted inside the bioreactor chamber, and washing fluid drain line of the bioreactor enable to wash and remove contaminations of the bioreactor chamber and lamps automatically without involving manual labour. Mounting of spray heads on the inner side of the bioreactor chamber wall opposite to the wall where light sources are mounted so that 4 spray heads are arranged symmetrically around each light source, that enables to wash almost complete surface of light sources. As a result of such arrangement of spray heads and optimal algorithm of washing the bioreactor 1 zones due to solenoid valves 7, not only the light source washing time is reduced, but also any possibility of infecting the bioreactor chamber with undesired bacteria is excluded.

The said solutions enable to improve the apparatus productivity and target product quality due to preventing from infecting the culture with other algae spores.

### The best example of the method implementation

The claimed method for producing microalgal suspension, in particular, food chlorella is implemented as follows.

The medium is preliminarily prepared using the program-controlled dosing pumps. The medium includes the following elements: N, P, Fe, C 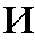 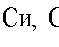 , Co, on a per 1000 ml of water bases comprises 0.14 ml of ammonium nitrate (34% solution), 0.10 ml of ammophos (15% solution), 0.15 ml of ferrous chloride (1% solution), 0.10 ml of cobaltous nitrate (0.1% solution), 0.10 ml of copper sulphate (0.1% solution).

Then, the bioreactor chamber is filled with stock chlorella suspension up to the necessary volume and the required volume of medium with the above composition is added. The culture mixture is illuminated in the automatic bioreactor within four successive cycles, each of them consisting of 10 hours of illumination and subsequent 2 hours of no illumination with artificial light sources being Off, and during illumination period the bioreactor temperature is maintained withing the range of 26-30°C, and during the period of no illumination - within the range of 24-2°C, that corresponds to developmental biology and promotes intensive growth and multiplication of microalgae cells. At that cultivation the culture reaches optical density of 1.4-1.8 D (440) (specified optical density). The cultivation is carried out subject to strict sanitary requirements for food products.

The produced suspension is poured from the bioreactor to the ready suspension tank, where chlorella is naturally precipitated within 30 days using natural diffused light, i.e. at normal lighting without artificial light sources.

Upon completion of microalgae cultivation cycles, the light sources are washed, washing fluid is drained.

All the processes, namely, washing fluid draining, filling the bioreactor chamber with stock culture and medium, controlling the illumination cycles, maintaining the temperature conditions, dosing of carbon dioxide and cyclic mixing are performed automatically by the devices controlled by the programmable controller.

The produced suspension is poured from the bioreactor to the food chlorella natural precipitation transparent tank, where chlorella is naturally precipitated within 30 days using natural diffused light, i.e. at normal lighting without artificial light sources.

The best embodiment of the claimed group of inventions, with illumination optical density within the range (1.4-1.8) D, enables to produce ready product in 48 hours, 11 of which comprises 7-10 grams of microalgal wet mass, that ensures high quality of ready product. The known methods for microalgae cultivation enable to produce 3-5 g/l.

The claimed method for cultivating microalgal biomass enables to produce in the claimed device the high-quality ready product which could be used in food, microbiological and pharmaceutical industry.

## Claims

1. The method for cultivating microalgal biomass including mineral medium preparation, adding of microalgae strain stock culture, filling of the produced culture mixture into the system of successively mounted bioreactors in the form of light-transmitting chambers horizontally oriented, illumination of the culture mixture by vertically arranged artificial light sources, extraction of the produced suspension to the natural precipitation tank and subsequent extraction of the produced precipitated biomass as a target product, **characterized in that**
- prepared mineral medium has the following composition:
ammonium nitrate (34% solution) 0.14 ml
ammophos (15% solution) 0.10 ml
ferrous chloride (1% solution) 0.15 ml
cobaltous nitrate (0.1% solution) 0.10 ml
copper sulphate (0.1 % solution) 0.10 ml
potable water 1000 ml
- cultivating the biomass in a single bioreactor chamber in the shape of a vertically oriented parallelepiped,
- illuminating the culture mixture by artificial light sources mounted on the inner side of one of the broad walls of the bioreactor chamber in horizontal rows throughout the height of the bioreactor chamber,
- illuminating the culture mixture cyclically,
- during all the cycles of microalgae cultivation maintaining pH value of the culture mixture in the range of 8.5-9.5 by the addition thereto of a lactic acid bacteria-containing solution at the beginning of each light cycle in an amount of 1-3 ml per litre of culture mixture, said solution having a pH value selected in a range of 4.0-5.0.

2. Method according to claim 1, **characterized in that** illuminating the culture mixture by artificial light sources within 4 light cycles, each of them consisting of 10 hours of illumination and subsequent 2 hours of no illumination, during illumination period the culture mixture temperature is maintained withing the range of 26-30°C, and during the period of no illumination
- within the range of 24-26°C.

3. Method according to claims 1, 2, **characterized in that** the culture mixture is filled into the bioreactor chamber above any horizontal row of light sources by a quantity equal to half of their center-to-center distance heightwise.

4. The microalgal biomass cultivation apparatus comprising successively mounted bioreactors in the form of transparent horizontal chambers, equipped with mixing means, connected to the ready microalgal biomass tank by a rundown line, the lactic-acid bacteria bioreactor connected at the outlet with microalgae bioreactors, medium preparation unit connected at the outlet with the microalgae bioreactors and lactic-acid bacteria solution bioreactor, artificial light sources in the form of electric lamps equipped with the cooling system, spray heads connected with the washing fluid preparation system, pumps and shutoff and control devices, **characterized in that**
- microalgae bioreactor chamber is in the shape of a vertically oriented transparent parallelepiped,
- artificial light sources are mounted on the inner side of one of the broad walls of the chamber in horizontal η rows perpendicular to it,
- spray heads are mounted on the inner side of the opposite wall of the bioreactor chamber so that 4 spray heads are arranged symmetrically around each artificial light source.

5. The apparatus according to claim 4, **characterized in that** it is equipped with programmable automatic system for control and monitoring the set parameters of temperature, volume, dosage, loading, unloading of solution and ready product.

6. The apparatus according to claim 4, **characterized in that** it comprises two or more bioreactors.
